(19) Europäisches Patentamt
European Patent Office
Office européen des brevets

(11) Veröffentlichungsnummer: **0 557 843 A2**

# (12) EUROPÄISCHE PATENTANMELDUNG

(21) Anmeldenummer: **93102326.1**

(22) Anmeldetag: **15.02.93**

(51) Int. Cl.$^5$: **C07D 401/12**, A61K 31/44

(30) Priorität: **27.02.92 DE 4206045**

(43) Veröffentlichungstag der Anmeldung:
**01.09.93 Patentblatt 93/35**

(84) Benannte Vertragsstaaten:
**AT BE CH DE DK ES FR GB GR IE IT LI LU MC NL PT SE**

(71) Anmelder: **BAYER AG**

**D-51368 Leverkusen(DE)**

(72) Erfinder: **Hanko, Rudolf, Dr.**
**Schillerstrasse 23**
**W-4000 Düsseldorf(DE)**
Erfinder: **Hübsch, Walter, Dr.**
**Am Eckbusch 43/50**
**W-5600 Wuppertal(DE)**
Erfinder: **Dressel, Jürgen, Ph. D.**
**Claudiusweg 9**
**W-5600 Wuppertal(DE)**
Erfinder: **Fey, Peter, Dr.**
**Am Eickhof 23**
**W-5600 Wuppertal(DE)**
Erfinder: **Krämer, Thomas, Dr.**
**In den Birken 92a**
**W-5600 Wuppertal(DE)**
Erfinder: **Müller, Ulrich E., Dr.**
**Claudiusweg 9**
**W-5600 Wuppertal(DE)**
Erfinder: **Müller-Gliemann, Matthias, Dr.**
**Laibacher Strasse 10**
**W-5650 Solingen(DE)**
Erfinder: **Beuck, Martin, Dr.**
**Trills 7**
**W-4006 Erkrath 2(DE)**
Erfinder: **Kazda, Stanislav, Prof. Dr.**
**Gellertweg 18**
**W-5600 Wuppertal(DE)**
Erfinder: **Hirth-Dietrich, Claudia, Dr.**
**Claudiusweg 9**
**W-5600 Wuppertal(DE)**
Erfinder: **Knorr, Andreas, Dr.**
**Trillser Graben 10**
**W-4006 Erkrath 2(DE)**
Erfinder: **Stasch, Johannes-Peter, Dr.**
**Schneewittchenweg 37**
**W-5600 Wuppertal(DE)**
Erfinder: **Wohlfeil, Stefan, Dr.**
**Tucherweg 25**
**W-4010 Hilden(DE)**
Erfinder: **Yalkinoglu, Özkan**
**Neuer Weg 21**
**W-5600 Wuppertal(DE)**

## (54) Sulfonylbenzyl-substituierte Pyridone als Arzneimittel.

(57) Sulfonylbenzyl-substituierte Pyridone können hergestellt werden indem man Pyridone mit Sulfonylbenzyl-Verbindungen umsetzt. Die Sulfonylbenzyl-substituierten Pyridonen können als Wirkstoffe in Arzneimitteln eingesetzt werden, insbesondere zur Behandlung von arterieller Hypertonie und Atherosklerose.

EP 0 557 843 A2

Die vorliegende Erfindung betrifft Sulfonylbenzyl-substituierte Pyridone, ein Verfahren zu ihrer Herstellung sowie ihre Verwendung in Arzneimitteln, insbesondere als blutdrucksenkendes und anti-atherosklerotisches Mittel.

Es ist bekannt, daß Renin, ein proteolytisches Enzym, in vivo vom Angiotensinogen das Dekapeptid Angiotensin I abspaltet, welches wiederum in der Lunge, den Nieren oder anderen Geweben zu dem blutdrucksteigernden Oktapeptid Angiotensin II abgebaut wird. Die verschiedenen Effekte des Angiotensin II, wie beispielsweise Vasokonstriktion, $Na^+$-Retention in der Niere, Aldosteronfreisetzung in der Nebenniere und Tonuserhöhung des sympathischen Nervensystems wirken synergistisch im Sinne einer Blutdruckerhöhung.

Darüber hinaus besitzt Angiotensin II die Eigenschaft, das Wachstum und die Vermehrung von Zellen wie beispielsweise von Herzmuskelzellen und glatten Muskelzellen zu fördern, wobei diese bei verschiedenen Krankheitszuständen (z.B. Hypertonie, Atherosklerose und Herzinsuffizienz) vermehrt wachsen und proliferieren.

Ein möglicher Ansatz zum Eingriff in das Renin-Angiotensin-System (RAS) ist neben der Inhibierung der Reninaktivität die Hemmung der Aktivität des Angiotensin-Konversionsenzyms (ACE) sowie die Blockade von Angiotensin II-Rezeptoren.

Die vorliegende Erfindung betrifft Sulfonylbenzyl-substituierte Pyridone der allgemeinen Formel (I)

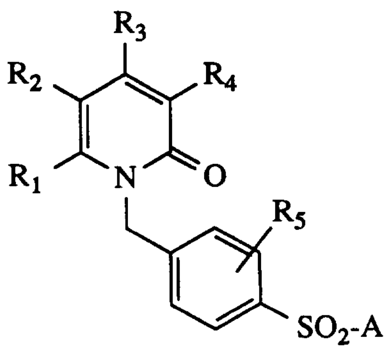

(I),

in welcher

$R^1$ für geradkettiges oder verzweigtes Alkyl mit bis zu 8 Kohlenstoffatomen steht, das gegebenenfalls durch Cycloalkyl mit 3 bis 6 Kohlenstoffatomen, Hydroxy oder durch geradkettiges oder verzweigtes Alkoxy mit bis zu 6 Kohlenstoffatomen substituiert ist, oder
für Cycloalkyl mit 3 bis 6 Kohlenstoffatomen steht,

$R^2$, $R^3$ und $R^4$ gleich oder verschieden sind und für Wasserstoff, Cyano oder geradkettiges oder verzweigtes Perfluoralkyl mit bis zu 8 Kohlenstoffatomen stehen, oder
für geradkettiges oder verzweigtes Alkyl mit bis zu 8 Kohlenstoffatomen stehen, das gegebenenfalls bis zu 2-fach gleich oder verschieden durch Hydroxy, Halogen, Carboxy, geradkettiges oder verzweigtes Alkoxy oder Alkoxycarbonyl mit jeweils bis zu 6 Kohlenstoffatomen, oder durch Phenyl, Phenoxy oder durch einen 5- bis 7-gliedrigen, gesättigten oder ungesättigten Heterocyclus mit bis zu 3 Heteroatomen substituiert ist, wobei die Cyclen ihrerseits bis zu 2-fach gleich oder verschieden durch Trifluormethyl, Trifluormethoxy, Halogen, Nitro, Cyano, Hydroxy, Hydroxymethyl oder durch geradkettiges oder verzweigtes Alkyl oder Alkoxy mit jeweils bis zu 6 Kohlenstoffatomen substituiert sein können, oder
für geradkettiges oder verzweigtes Acyl oder Alkoxycarbonyl mit jeweils bis zu 8 Kohlenstoffatomen, Benzyloxycarbonyl oder Carboxy stehen, oder
für Phenyl stehen, das gegebenenfalls bis zu 3-fach gleich oder verschieden durch Halogen, Nitro, Cyano, Hydroxy, Hydroxymethyl, Trifluormethyl, Trifluormethoxy oder durch geradkettiges oder verzweigtes Alkyl oder Alkoxy mit jeweils bis zu 6 Kohlenstoffatomen substituiert ist, oder für eine Gruppe der Formel $-CO-NR^6R^7$ stehen,
worin

$R^6$ und $R^7$ gleich oder verschieden sind und Wasserstoff, geradkettiges oder verzweigtes Alkyl mit bis zu 8 Kohlenstoffatomen oder Aryl oder Aralkyl mit jeweils 6 bis 10 Kohlenstoff-

atomen bedeuten,

$R^5$ für Wasserstoff, Halogen oder geradkettiges oder verzweigtes Alkyl mit bis zu 8 Kohlenstoffatomen, oder
für geradkettiges oder verzweigtes Perfluoralkyl mit bis zu 6 Kohlenstoffatomen steht, oder
für eine Gruppe der Formel -OX steht,
worin

X Wasserstoff, Benzyl, eine Hydroxyschutzgruppe oder geradkettiges oder verzweigtes Alkyl mit bis zu 8 Kohlenstoffatomen bedeutet,

A für einen über das Stickstoffatom gebundenen 3 bis 8-gliedrigen, gesättigten Heterocyclus mit bis zu 2 weiteren Heteroatomen aus der Reihe S, N oder O steht und der gegebenenfalls bis zu 2-fach gleich oder verschieden durch Perfluoralkyl mit bis zu 5 Kohlenstoffatomen oder durch einen Rest der Formel
$-SO_3H$ ,

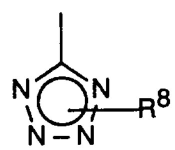 ,

$-CO-R^9$ oder

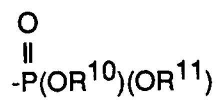

substituiert ist,
worin

$R^8$ Wasserstoff, geradkettiges oder verzweigtes Alkyl mit bis zu 6 Kohlenstoffatomen oder Triphenylmethyl bedeutet,

$R^9$ Hydroxy, geradkettiges oder verzweigtes Alkoxy mit bis zu 8 Kohlenstoffatomen, Phenoxy, Benzyloxy oder eine Gruppe der Formel $-NR^{12}R^{13}$ bedeutet,
worin

$R^{12}$ und $R^{13}$ gleich oder verschieden sind und Wasserstoff oder geradkettiges oder verzweigtes Alkyl mit bis zu 6 Kohlenstoffatomen oder Phenyl bedeuten,

$R^{10}$ und $R^{11}$ gleich oder verschieden sind und Wasserstoff, geradkettiges oder verzweigtes Alkyl mit bis zu 8 Kohlenstoffatomen oder Phenyl bedeuten,

und deren Salze.

Die erfindungsgemäßen Sulfonylbenzyl-substituierten Pyridone können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der Sulfonylbenzyl substituierten Pyridone können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen, sein. Besonders bevorzugt sind z.B. Natrium-, Kalium-, Magnesium- oder Calciumsalze, sowie Ammoniumsalze, die abgeleitet sind von Ammoniak oder organischen Aminen wie beispielsweise Ethylamin, Di- bzw. Triethylamin, Di- bzw. Triethanolamin, Dicyclohexylamin, Dimethylaminoethanol, Arginin, Lysin oder Ethylendiamin.

Die erfindungsgemäßen Verbindungen können in stereoisomeren Formen, entweder als Enantiomere oder als Diastereomere, existieren. Die Erfindung betrifft sowohl die Enantiomeren oder Diastereomeren als auch deren jeweiligen Mischungen. Die Racemformen lassen sich ebenso wie die Diastereomeren in bekannter Weise in die stereoisomer einheitlichen Bestandteile trennen [vgl. E.L. Eliel, Stereochemistry of

Carbon Compounds, McGraw Hill, 1962].

Heterocyclus unter den Definitionen von $R^2$, $R^3$ und $R^4$ steht im allgemeinen für einen 5- bis 7-gliedrigen, bevorzugt 5- bis 6-gliedrigen, gesättigten oder ungesättigten Ring, der als Heteroatome bis zu 2 Sauerstoff-, Schwefel- und/oder Stickstoffatome enthalten kann. Bevorzugt sind 5- und 6-gliedrige Ringe mit einem Sauerstoff-, Schwefel und/oder bis zu 2 Stickstoffatomen. Bevorzugt werden genannt: Thienyl, Furyl, Pyrrolyl, Pyrazolyl, Pyridyl, Pyrimidyl, Pyrazinyl, Pyridazinyl, Thiazolyl, Oxazolyl, Imidazolyl, Pyrrolidinyl, Piperidinyl, Piperazinyl oder Tetrazolyl.

Ein über das Stickstoffatom gebundener, 3- bis 8-gliedriger gesättigter Heterocyclus, der außerdem als Heteroatome bis zu 2 Sauerstoff-, Schwefel- und/oder Stickstoffatome enthalten kann, steht im allgemeinen für Azetidinyl, Piperidyl, Morpholinyl, Piperazinyl oder Pyrrolidinyl. Bevorzugt sind 5- und 6-gliedrige Ringe mit einem Sauerstoff- und/oder bis zu 2-Stickstoffatomen, wie beispielsweise Piperidyl, Morpholinyl oder Pyrrolidinyl. Besonders bevorzugt sind Piperidyl und Pyrrolidinyl.

Bevorzugt sind Verbindungen der allgemeinen Formel (I),
in welcher

$R^1$ für geradkettiges oder verzweigtes Alkyl mit jeweils bis zu 6 Kohlenstoffatomen steht, das gegebenenfalls durch Cyclopropyl, Cyclopentyl, Cyclohexyl, Hydroxy oder durch geradkettiges oder verzweigtes Alkoxy mit bis zu 4 Kohlenstoffatomen substituiert ist,
oder
für Cyclopropyl, Cyclopentyl oder Cyclohexyl steht,

$R^2$, $R^3$ und $R^4$ gleich oder verschieden sind und für Wasserstoff, Cyano oder für geradkettiges oder verzweigtes Perfluoralkyl mit bis zu 6 Kohlenstoffatomen stehen, oder
für geradkettiges oder verzweigtes Alkyl mit bis zu 6 Kohlenstoffatomen stehen, das durch Hydroxy, Fluor, Chlor, Brom, Carboxy, geradkettiges oder verzweigtes Alkoxy oder Alkoxycarbonyl mit jeweils bis zu 4 Kohlenstoffatomen, oder durch Phenyl, Phenoxy oder Thienyl substituiert ist, wobei die Cyclen ihrerseits durch Trifluormethoxy, Trifluormethyl, Hydroxymethyl, Fluor, Chlor, Brom oder durch geradkettiges oder verzweigtes Alkyl oder Alkoxy mit jeweils bis zu 6 Kohlenstoffatomen substituiert sein können, oder
für geradkettiges oder verzweigtes Acyl oder Alkoxycarbonyl mit jeweils bis zu 6 Kohlenstoffatomen, Benzyloxycarbonyl oder Carboxy stehen, oder für Phenyl stehen, das gegebenenfalls bis zu 2-fach gleich oder verschieden durch Fluor, Chlor, Brom, Trifluormethyl, Trifluormethoxy, Hydroxymethyl oder durch geradkettiges oder verzweigtes Alkyl oder Alkoxy mit jeweils bis zu 4 Kohlenstoffatomen substituiert ist, oder
für eine Gruppe der Formel $-CONR^6R^7$ stehen,
worin

$R^6$ und $R^7$ gleich oder verschieden sind und Wasserstoff, geradkettiges oder verzweigtes Alkyl mit bis zu 6 Kohlenstoffatomen, Phenyl oder Benzyl bedeuten,

$R^5$ für Wasserstoff, Fluor, Chlor, Brom, geradkettiges oder verzweigtes Alkyl mit bis zu 6 Kohlenstoffatomen, oder
für geradkettiges oder verzweigtes Perfluoralkyl mit bis zu 4 Kohlenstoffatomen steht,
oder
für eine Gruppe der Formel -OX steht,
worin

X Wasserstoff, Benzyl, Acetyl oder geradkettiges oder verzweigtes Alkyl mit bis zu 6 Kohlenstoffatomen bedeutet,

A für über das Stickstoffatom gebundenes Piperidyl, Pyrrrolidinyl oder Morpholinyl steht, die gegebenenfalls durch Trifluormethyl oder durch einen Rest der Formel
$-SO_3H$ ,

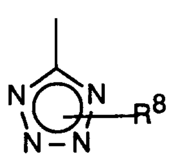
,

$-CO-R^9$ oder

$$-\overset{\displaystyle O}{\overset{\|}{P}}(OR^{10})(OR^{11})$$

substituiert sind,
worin

$R^8$ Wasserstoff, geradkettiges oder verzweigtes Alkyl mit bis zu 4 Kohlenstoffatomen oder Triphenylmethyl bedeutet,

$R^9$ Hydroxy, geradkettiges oder verzweigtes Alkoxy mit bis zu 6 Kohlenstoffatomen, Phenoxy, Benzyloxy oder eine Gruppe der Formel $-NR^{12}R^{13}$ bedeutet,
worin

$R^{12}$ und $R^{13}$ gleich oder verschieden sind und Wasserstoff oder geradkettiges oder verzweigtes Alkyl mit bis zu 4 Kohlenstoffatomen bedeuten,

$R^{10}$ und $R^{11}$ gleich oder verschieden sind und Wasserstoff, geradkettiges oder verzweigtes Alkyl mit bis zu 6 Kohlenstoffatomen oder Phenyl bedeuten,

und deren Salze.

Besonders bevorzugt sind Verbindungen der allgemeinen Formel (I),
in welcher

$R^1$ für geradkettiges oder verzweigtes Alkyl mit bis zu 4 Kohlenstoffatomen steht, das gegebenenfalls durch Cyclopropyl substituiert ist oder für Cyclopropyl steht,

$R^2$, $R^3$ und $R^4$ gleich oder verschieden sind und für Wasserstoff, Cyano oder für geradkettiges oder verzweigtes Perfluoralkyl mit bis zu 4 Kohlenstoffatomen stehen,
für geradkettiges oder verzweigtes Alkyl mit bis zu 4 Kohlenstoffatomen stehen, das durch Hydroxy oder geradkettiges oder verzweigtes Alkoxy oder Alkoxycarbonyl mit jeweils bis zu 3 Kohlenstoffatomen substituiert ist, oder für geradkettiges oder verzweigtes Acyl oder Alkoxycarbonyl mit jeweils bis zu 4 Kohlenstoffatomen, Benzyloxycarbonyl oder Carboxy stehen, oder
für eine Gruppe der Formel $-CONR^6R^7$ stehen,
worin

$R^6$ und $R^7$ gleich oder verschieden sind und Wasserstoff, geradkettiges oder verzweigtes Alkyl mit bis zu 4 Kohlenstoffatomen, Phenyl oder Benzyl bedeuten,

$R^5$ für Wasserstoff, Fluor, Chlor, geradkettiges oder verzweigtes Alkyl mit bis zu 4 Kohlenstoffatomen, oder
für geradkettiges oder verzweigtes Perfluoralkyl mit bis zu 3 Kohlenstoffatomen steht, oder
für eine Gruppe der Formel -OX steht,
worin

X Wasserstoff, Benzyl, Acetyl oder geradkettiges oder verzweigtes Alkyl mit bis zu 6 Kohlenstoffatomen bedeutet,

A für über das Stickstoffatom gebundenes Piperidyl oder Pyrrolidinyl steht, die gegebenenfalls durch Trifluormethyl oder durch einen Rest der Formel
$-SO_3H$ ,

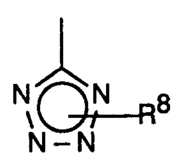 ,

$-CO-R^9$ oder

$$-\overset{\displaystyle O}{\overset{\|}{P}}(OR^{10})(OR^{11})$$

substituiert sind,
worin

$R^8$ Wasserstoff, Methyl, Ethyl oder Triphenylmethyl bedeutet,

$R^9$ Hydroxy, geradkettiges oder verzweigtes Alkoxy mit bis zu 4 Kohlenstoffatomen, Phenoxy, Benzyloxy oder eine Gruppe der Formel $-NR^{12}R^{13}$ bedeutet,
worin

$R^{12}$ und $R^{13}$ gleich oder verschieden sind und Wasserstoff oder geradkettiges oder verzweigtes Alkyl mit bis zu 3 Kohlenstoffatomen bedeuten
und

$R^{10}$ und $R^{11}$ gleich oder verschieden sind und Wasserstoff, geradkettiges oder verzweigtes Alkyl mit bis zu 4 Kohlenstoffatomen oder Phenyl bedeuten,

und deren Salze.

Außerdem wurde ein Verfahren zur Herstellung der erfindungsgemäßen Verbindungen der allgemeinen Formel (I) gefunden, dadurch gekennzeichnet, daß man Pyridone der allgemeinen Formel (II)

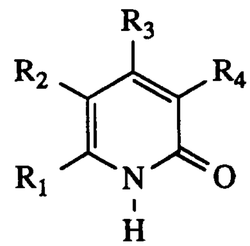

(II),

in welcher

$R^1$, $R^2$, $R^3$ und $R^4$ die oben angegebene Bedeutung haben,

mit Verbindungen der allgemeinen Formel (III)

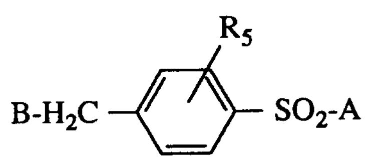

(III),

in welcher

$R^5$ und A die oben angegebene Bedeutung haben
und

B für Halogen, vorzugsweise für Brom steht,

in organischen Lösemitteln und in Anwesenheit einer Base und gegebenenfalls eines Katalysators umsetzt, und im Fall, daß $R^8 \neq$ Wasserstoff eine Alkylierung anschließt und im Fall der Säuren ($R^9$ = OH) die entsprechenden Ester verseift
und im Fall der Ester oder Amide, gegebenenfalls über eine aktivierte Carbonsäurestufe, eine Veresterung bzw. Amidierung anschließt
und die Substituenten $R^2$, $R^3$, $R^4$ und $R^5$ nach üblichen Methoden variiert.

Das erfindungsgemäße Verfahren kann durch folgendes Formelschema beispielhaft erläutert werden:

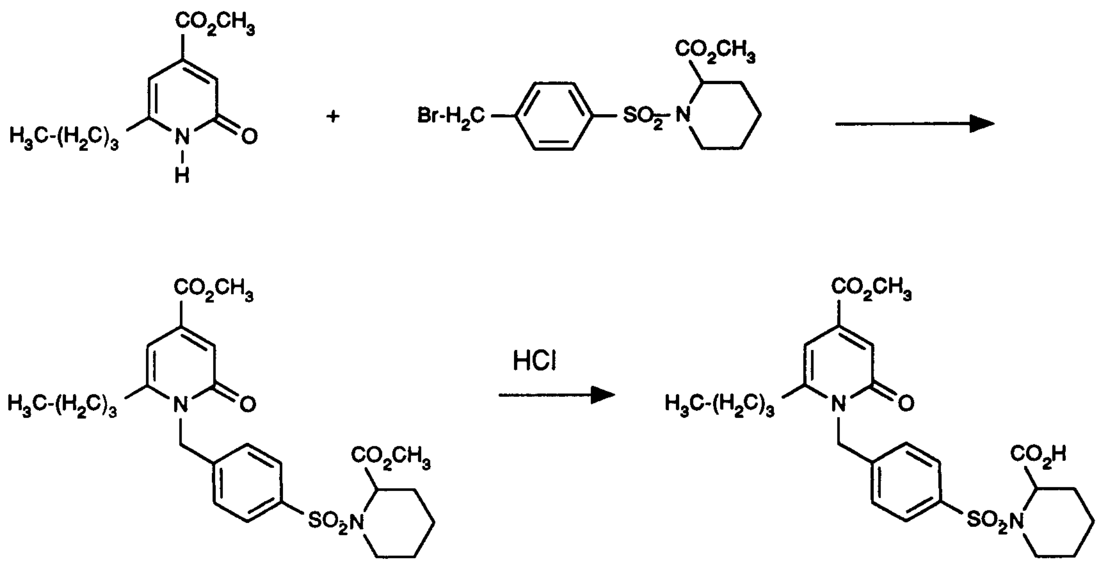


Als Lösemittel für das Verfahren eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, 1,2-Dimethoxyethan oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt sind Tetrahydrofuran und 1,2-Dimethoxyethan.

Als Basen für das erfindungsgemäße Verfahren können im allgemeinen anorganische oder organische Basen eingesetzt werden. Hierzu gehören vorzugsweise Alkalihydroxide oder Erdalkalihydroxide wie zum Beispiel Natriumhydroxid, Kaliumhydroxid oder Lithiumhydroxid, Bariumhydroxid, Alkali- oder Erdalkalicarbonate wie Natriumcarbonat, Kaliumcarbonat, Calciumcarbonat oder Caesiumcarbonat, oder Alkali- oder Erdalkalialkoholate wie Natrium- oder Kaliummethanolat oder Kalium-tert.butylat, oder Lithiumdiisopropylamid (LDA), oder organische Amine (Trialkyl($C_1$-$C_6$)amine) wie Triethylamin, oder Heterocyclen wie 1,4-Diazabicyclo[2.2.2]octan (DABCO), 1,8-Diazabicyclo[5.4.0]-undec-7-en (DBU), Pyridin, Diaminopyridin, Methylpiperidin oder Morpholin. Es ist auch möglich als Basen Alkalimetalle, wie Natrium oder deren Hydride wie Natriumhydrid einzusetzen. Bevorzugt sind Kaliumcarbonat, Natriumhydrid, Kalium-tert.-butylat und Caesiumcarbonat.

Im allgemeinen setzt man die Base in einer Menge von 0,05 mol bis 10 mol, bevorzugt von 1 mol bis 2 mol bezogen auf 1 mol der Verbindung der Formel (III) ein.

Das erfindungsgemäße Verfahren wird im allgemeinen in einem Temperaturbereich von -100 °C bis +100 °C, bevorzugt von 0 °C bis 40 °C durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Abspaltung der Triphenylmethylgruppe erfolgt mit Essigsäure oder Trifluoressigsäure und Wasser oder einem der oben aufgeführten Alkohole oder mit wäßriger Salzsäure in Anwesenheit von Aceton oder ebenfalls mit Alkoholen.

Die Abspaltung erfolgt im allgemeinen in einem Temperaturbereich von 0 °C bis 150 °C, vorzugsweise von 20 °C bis 100 °C und Normaldruck.

Als Katalysatoren eignen sich Kalium- oder Natriumiodid, bevorzugt Natriumiodid.

Die Alkylierung erfolgt im allgemeinen mit Alkylierungsmitteln wie beispielsweise ($C_1$-$C_6$)-Alkylhalogeniden, Sulfonsäurestern oder substituierten oder unsubstituierten ($C_1$-$C_6$)-Dialkyl- oder ($C_1$-$C_6$)-Diarylsulfonate, vorzugsweise Methyliodid oder Dimethylsulfat.

Die Alkylierung erfolgt im allgemeinen in einem der oben aufgeführten Lösemitteln, vorzugsweise in Dimethylformamid in einem Temperaturbereich von 0 °C bis +70 °C, vorzugsweise von 0 °C bis +30 °C und Normaldruck.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kalium-tert.butanolat. Besonders bevorzugt werden Natriumhydroxid, Kaliumhydroxid oder Lithiumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid, oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen. Bevorzugt sind Tetrahydrofuran und Methanol.

Die Verseifung kann gegebenenfalls auch mit Säuren wie beispielsweise Trifluoressigsäure, Essigsäure, Chlorwasserstoffsäure, Bromwasserstoffsäure, Methansulfonsäure, Schwefelsäure oder Perchlorsäure, bevorzugt mit Trifluoressigsäure erfolgen.

Die Verseifung wird im allgemeinen in einem Temperaturbereich von 0 °C bis +100 °C, bevorzugt von +20 °C bis +80 °C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters, eingesetzt. Besonders bevorzugt verwendet man molare Mengen der Reaktanden.

Die Verseifung von tert.-Butylestern erfolgt im allgemeinen mit Säuren, wie beispielsweise Salzsäure oder Trifluoressigsäure, in Anwesenheit eines der oben angegebenen Lösemitteln und/oder Wasser oder deren Gemische, vorzugsweise mit Dioxan oder Tetrahydrofuran.

Die Amidierung und die Sulfonamidierung erfolgen im allgemeinen in einem der oben aufgeführten Lösemitteln, vorzugsweise in Tetrahydrofuran oder Dichlormethan.

Die Amidierung und die Sulfonamidierung können gegebenenfalls über die aktivierte Stufe der Säurehalogenide, die aus den entsprechenden Säuren durch Umsetzung mit Thionylchlorid, Phosphortrichlorid, Phosphorpentachlorid, Phosphortribromid oder Oxalylchlorid hergestellt werden können, verlaufen.

Die Amidierung und die Sulfonamidierung erfolgt im allgemeinen in einem Temperaturbereich von -20 °C bis +80 °C, vorzugsweise von -10 °C bis +30 °C und Normaldruck.

Als Basen eignen sich dafür neben den oben aufgeführten Basen vorzugsweise Triethylamin und/oder Dimethylaminopyridin, DBU oder DABCO.

Die Base wird in einer Menge von 0,5 mol bis 10 mol, bevorzugt von 1 mol bis 2 mol, bezogen auf 1 mol der entsprechenden Säure oder Esters, eingesetzt.

Als säurebindende Mittel für die Sulfonamidierung können Alkali- oder Erdalkalicarbonate wie Natriumcarbonat, Kaliumcarbonat, Alkali- oder Erdalkalihydroxide wie beispielsweise Natrium- oder Kaliumhydroxid, oder organische Basen wie Pyridin, Triethylamin, N-Methylpiperidin, oder bicyclische Amidine wie 1,5-Diazabicyclo[3.4.0]-nonene-5 (DBN) oder 1,5-Diazabicyclo[3.4.0]undecene-5 (DBU) eingesetzt werden. Bevorzugt ist Kaliumcarbonat.

Als Dehydratisierungsreagenzien eignen sich Carbodiimide wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphonsäureanhydrid oder Isobutylchloroformat oder Benzotriazolyloxy-tris-(dimethylamino)phosphonium-hexafluorophosphat oder Phosphonsäurediphenylesteramid oder Methansulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid [vgl. J.C. Sheehan, S.L. LEdis, J. Am. Chem. Soc. 95, 875 (1973); F.E. Frerman et al., J. Biol. Chem. 225, 507 (1982) und N.B. Benoton, K. Kluroda, Int. Pept. Prot. Res. 13, 403 (1979), 17, 187 (1981)].

Die säurebindenden Mittel und Dehydratisierungsreagenzien werden im allgemeinen in einer Menge von 0,5 bis 3 mol, bevorzugt von 1 bis 1,5 mol, bezogen auf 1 mol der entsprechenden Carbonsäuren, eingesetzt.

Die Verbindungen der allgemeinen Formel (II) sind teilweise bekannt [vgl. z.B. DE 3 406 329 A1, R.P. Mariella, R. Stansfield, J.Am. Chem. Soc. 73, 1368 (1951) und O. Isler et al., Helv. Chim. Acta 38, 1033 (1955) Bull. Soc. Chim. Fr. 687 (1958)] oder neu und können dann in Analogiemethoden zu den oben zitierten Publikationen hergestellt werden.

Die Verbindungen der allgemeinen Formel (III) sind neu und können hergestellt werden, indem man substituierte Benzylsulfonsäurechloride der allgemeinen Formel (IV)

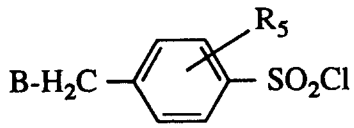

(IV),

in welcher

B und $R^5$ die oben angegebene Bedeutung haben

mit Verbindungen der allgemeinen Formel (V)

H-A (V),

in welcher

A die oben angegebene Bedeutung hat,

in einem der oben angegebenen Lösemittel und Basen, vorzugsweise in Dichlormethan mit Triethylamin umsetzt.

Im allgemeinen wird die Umsetzung bei Normaldruck durchgeführt. Es ist aber auch möglich, bei Unterdruck oder bei Überdruck zu arbeiten (z.B. von 0,5 bis 5 bar).

Bei der Durchführung der Umsetzung wird die Base im allgemeinen in einer Menge von 1 bis 3 mol, bevorzugt von 1 bis 1,5 mol bezogen auf 1 mol der Verbindungen der allgemeinen Formel (IV), eingesetzt. Besonders bevorzugt verwendet man molare Mengen der Reaktanden.

Die Umsetzung erfolgt im allgemeinen in einem Temperaturbereich von -40 ° C bis +40 ° C, vorzugsweise von -30 ° C bis 0 ° C und unter Normaldruck.

Die Verbindungen der allgemeinen Formeln (IV) und (V) sind bekannt oder können nach üblicher Methode hergestellt werden.

Die erfindungsgemäßen Verbindungen der allgemeinen Formel (I) zeigen ein nicht vorhersehbares, wertvolles pharmakologisches Wirkspektrum.

Die erfindungsgemäßen Verbindungen besitzen eine spezifische A II-antagonistische Wirkung, da sie die Bindung von Angiotensin II an A II-Rezeptoren hemmen. Sie unterdrücken die vasokonstriktorischen und Aldosteronsekretions-stimulierendenEffekte des Angiotensin II. Darüberhinaus inhibieren sie die Proliferation von glatten Muskelzellen.

Sie können deshalb in Arzneimitteln zur Behandlung der arteriellen Hypertonie und Atherosklerose eingesetzt werden. Darüberhinaus können sie zur Behandlung von coronaren Herzerkrankungen, Herzinsuffizienz, Störungen der Hirnleistung, ischämischen Gehirnerkrankungen, peripherer Durchblutungsstörungen, Funktionsstörungen der Niere und Nebenniere, bronchospastischen und vaskulär bedingten Erkrankungen der Atemwege, Natriumretention und Ödemen eingesetzt werden.

Darüber hinaus besitzen die Substanzen natriuretische und diuretische Wirkung. Diese Wirkung äußert sich in einer Ödemausschwemmung bei pathologischer Flüssigkeitsvermehrung kardialen und nicht kardialen Ursprungs.

Untersuchung auf die Hemmung der mit Agonisten induzierten Kontraktion

Kaninchen beiderlei Geschlechts werden durch Nackenschlag betäubt und entblutet, oder fallweise mit Nembutal (ca. 60 - 80 mg/kg i.v.) narkotisiert und durch Öffnung des Thorax getötet. Die Thoraxaorta wird entnommen, von anhaftendem Bindegewebe befreit, in 1,5 mm breite Ringsegmente geteilt und einzeln unter einer Anfangsbelastung von ca. 3,5 g in 10 ml Organbäder mit auf 37 ° C temperierter, Carbogenbegaster Krebs-Henseleit-Nährlösung folgender Zusammensetzung: 119 mmol/l NaCl; 2,5 mmol/l $CaCl_2$ x 2 $H_2O$; 1,2 mmol/l $KH_2PO_4$; 10 mmol/l Glucose; 4,8 mmol/l KCl; 1,4 mmol/l $MgSO_4$ x 7 $H_2O$ und 25 mmol/l $NaHCO_3$ verbracht.

Die Kontraktionen werden isometrisch durch Statham UC2-Zellen über Brückenverstärker (ifd Mülheim bzw. DSM Aalen) erfaßt und mittels A/D-Wandler (System 570, Keithley München) digitalisiert sowie ausgewertet. Die Durchführung von Agonistdosiswirkungskurven (DWK) erfolgt stündlich. Mit jeder DWK werden 3 bzw. 4 Einzelkonzentrationen im 4 min-Abstand in die Bäder appliziert. Nach Ende der DWK und darauffolgender Auswaschzyklen (16 mal jeweils ca. 5 sec/min mit der o.a. Nährlösung) schließt sich eine 28-minütige Ruhe- bzw. Inkubationsphase an, innerhalb derer die Kontraktionen in der Regel wieder den Ausgangswert erreichen.

Die Höhe der im Normalfall 3. DWK wird als Bezugsgröße für die Bewertung der in weiteren Durchgängen zu untersuchenden Testsubstanz verwendet, die bei den nachfolgenden DWK's in jeweils

steigender Dosierung mit Beginn der Inkubationszeit in die Bäder appliziert wird. Jeder Aortenring wird dabei ganztägig mit immer dem gleichen Agonisten stimuliert.

Agonisten und ihre Standardkonzentrationen (Applikationsvolumen pro Einzelgabe = 100 µl):

| | | |
|---|---|---|
| KCl | 22,7;32,7;42,7;52,7 | mmol/l |
| 1-Noradrenalin | $3x10^{-9}$;$3x10^{-8}$;$3x10^{-7}$;$3x10^{-6}$ | g/ml |
| Serotonin | $10^{-8}$;$10^{-7}$;$10^{-6}$;$10^{-5}$ | g/ml |
| B-HT 920 | $10^{-7}$;$10^{-6}$;$10^{-5}$ | g/ml |
| Methoxamin | $10^{-7}$;$10^{-6}$;$10^{-5}$ | g/ml |
| Angiotensin II | $3x10^{-9}$;$10^{-8}$;$3x10^{-8}$;$10^{-7}$ | g/ml |

Für die Berechnung der $IC_{50}$ (Konzentration bei der die zu untersuchende Substanz eine 50%ige Hemmung verursacht) wird der Effekt jeweils an der 3. = submaximalen Agonistkonzentration zugrundegelegt.

Die erfindungsgemäßen Verbindungen hemmen die durch Angiotensin II induzierte Kontraktion der isolierten Kaninchenaorta dosenabhängig. Die durch Kalium-Depolarisation oder andere Agonisten induzierte Kontraktion wurde nicht oder in hohen Konzentrationen nur schwach gehemmt.

**Tabelle A:**

**Hemmung der Gefäßkontraktion an isolierten Aortenringen von Kaninchen in vitro**

**$IC_{50}$ (nM) gegen Kontraktionen, induziert durch:**

| Bsp.Nr.: | AII |
|---|---|
| 6 | 280 |

Blutdruckmessungen an der Angiotensin II-infundierten Ratte

Männliche Wistar Ratten (Moellegaard, Kopenhagen, Dänemark) mit einem Körpergewicht von 300 - 350 g, werden mit Thiopental (100 mg/kg i.p.) anästhesiert. Nach Tracheotomie wird in die Femoralarterie ein Katheter zur Blutdruckmessung und in die Femoralvenen ein Katheter für die Angiotensin II-Infusion und ein Katheter für die Substanzverabreichung eingeführt. Nach Gabe des Ganglienblockers Pentolinium (5 mg/kg i.v.) wird die Angiotensin II-Infusion (0,3 µg/kg/min) gestartet. Sobald die Blutdruckwerte ein stabiles Plateau erreicht haben, werden die Testsubstanzen entweder intravenös oder als Suspension bzw. Lösung in 0,5% Tylose oral verabreicht. Die Blutdruckveränderungen unter Substanzeinfluß sind als Mittelwerte ± SEM in der Tabelle angegeben.

Bestimmung der antihypertensiven Wirksamkeit bei wachen hypertensiven Ratten

Die orale antihypertensive Wirksamkeit der erfindungsgemäßen Verbindungen wurde an wachen Ratten mit chirurgisch induzierter unilateraler Nierenarterienstenose geprüft. Dazu wurde die rechte Nierenarterie mit einem Silberclip von 0,18 mm lichter Weite eingeengt. Bei dieser Hypertonieform ist die Plasmareninaktivität in den ersten sechs Wochen nach dem Eingriff erhöht.

Der arterielle Blutdruck diese Tiere wurde in definierten Zeitabständen nach Substanzgabe mit der "Schwanzmanschette" unblutig gemessen. Die zu prüfenden Substanzen wurden aufgeschwemmt in einer

Tylosesuspension intragastral ("oral") per Schlundsonde in verschiedenen Dosen appliziert. Die erfindungsgemäßen Verbindungen senken den arteriellen Blutdruck der Hochdruckratten in klinisch relevanter Dosierung.

Außerdem hemmen die erfindungsgemäßen Verbindungen die spezifische Bindung von radioaktivem Angiotensin II konzentrationsabhängig.

Interaktion der erfindungsgemäßen Verbindungen mit dem Angiotensin II-Rezeptor an Membranfraktionen der Nebennierenrinde (Rind)

Nebennierenrinden vom Rind (NNR), die frisch entnommen und sorgfältig von Mark von Kapsel befreit sind, werden in Sucrose-Lösung (0,32 M) mit Hilfe eines Ultra-Turrax (Janke & Kunkel, Staufen i.B.) zu grobem Membran-Homogenat zerkleinert und in zwei Zentrifugationsschritten zu Membranfraktionen partiell aufgereinigt.

Die Untersuchungen zur Rezeptor-Bindung werden an partiell gereinigten Membranfraktionen boviner NNR mit radioaktivem Angiotensin II in einem Assay-Volumen von 0,25 ml durchgeführt, das im einzelnen die partiell gereinigten Membranen (50 - 80 µg), $^3$H-Angiotensin II (3-5 nM), Test-Pufferlösung (50 mM Tris, pH 7,2), 5 mM $MgCl_2$ sowie die zu untersuchenden Substanzen enthält. Nach einer Inkubationszeit von 60 min bei Raumtemperatur wird die nicht gebundene Radioaktivität der Proben mittels angefeuchteter Glasfaserfilter (Whatman GF/C) separiert und die gebundene Radioaktivität nach Waschung des Proteins mit eiskalter Pufferlösung (50 mM Tris/HCl, pH 7,4, 5% PEG 6000) in einem Szintillationscocktail spektrophotometrisch gemessen. Die Analyse der Rohdaten erfolgte mit Computer-Programmen zu $K_i$- bzw. $IC_{50}$-Werten ($K_i$: für die verwendete Radioaktivität korrigierte $IC_{50}$-Werte; $IC_{50}$-Werte: Konzentration bei der die zu untersuchende Substanz eine 50%ige Hemmung der spezifischen Bindung des Radioliganden bewirkt).

Untersuchung zur Inhibition der Proliferation glatter Muskelzellen durch die erfindungsgemäßen Verbindungen

Zur Feststellung der antiproliferativen Wirkung der Verbindungen werden glatte Muskelzellen verwendet, die aus den Aorten von Ratten oder Schweinen durch die Media-Explantat-Technik gewonnen werden [R. Ross, J. Cell. Biol. 50, 172, 1971]. Die Zellen werden in geeigneten Kulturschalen, in der Regel 24-Loch-Platten, ausgesät und für 2 - 3 Tage in Medium 199 mit 7,5% FCS und 7,5% NCS, 2 mM L-Glutamin und 15 mM HEPES, pH 7,4 in 5% $CO_2$ bei 37 °C kultiviert. Danach werden die Zellen durch Serumentzug für 2 - 3 Tage synchronisiert und sodann mit AII, Serum oder anderen Faktoren zum Wachstum angeregt. Gleichzeitig werden Testverbindungen zugesetzt. Nach 16 - 20 Stunden wird 1 µCi $^3$H-Thymidin zugefügt und nach weiteren 4 Stunden der Einbau dieser Substanz in die TCA-präzipitierbare DNA der Zellen bestimmt.

Prüfung auf natriuretische Wirkung

Nüchterne Wistar-Ratten werden mit Prüfsubstanz (suspendiert in Tylose-Lösung) oral behandelt. Anschließend wird in Diurese-Käfigen die Harnausscheidung über 6 Std. gesammelt. Die Konzentration von Natrium und Kalium im Harn wird flammenphotometrisch bestimmt.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nichttoxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu

verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Lösemittel:

a = $CH_2Cl_2/CH_3OH$ = 10:1
b = $CH_2Cl_2/CH_3OH/CH_3CO_2H$ = 10:1:0,5
c = $CH_2Cl_2/CH_3OH$ = 5:1

Ausgangsverbindungen

Beispiel I

6-Butyl-4-methoxycarbonyl-2-oxo-1,2-dihydropyridin

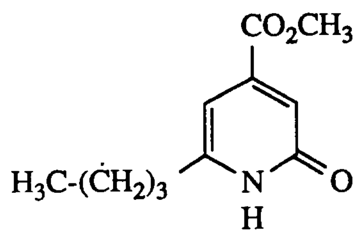

Zu einer Suspension von 29,25 g (0,15 mol) 6-Butyl-2-oxo-1,2-dihydro-isonicotinsäure in 200 ml Methanol tropft man unter Eiskühlung 12,5 ml (0,17 mol) Thionylchlorid und rührt über Nacht bei Raumtemperatur. Man engt zur Trockne ein und chromatographiert den Rückstand über 450 g Kieselgel (230-400 mesh) mit Dichlormethan → Dichlormethan/Methanol 10:1. Aus Dichlormethan, Ether, Petrolether, kristallisieren 29,6 g (94 %) farblose Kristalle vom Schmelzpunkt 106 °C.

Beispiel II

6-Butyl-pyrid-2(1H)-on

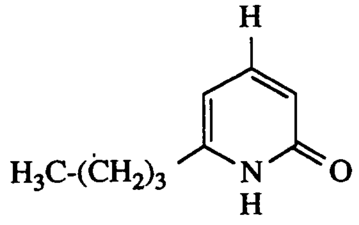

4,9 g (25 mmol) 6-Butyl-2-oxo-1,2-dihydro-isonicotinsäure werden mit 1,79 g (12,5 mmol) Kupfer-I-oxid in 50 ml Chinolin 1,5 h am Rückfluß gekocht (237 °C). Nach dem Abfiltrieren werden die flüchtigen Bestandteile im Vakuum abdestilliert (110 °C bei 17 mbar, danach 67 °C bei 9 mbar). Der Rückstand wird zweimal an Kieselgel mit Dichlormethan/Methanol (40:1) → (20:1) chromatographiert und das Produkt in

Petrolether ausgerührt.
Ausbeute: 1,95 g (52 %) bräunliche Kristalle vom Schmelzpunkt 68 ° C

Beispiel III

4-(Brommethyl)benzol-sulfochlorid

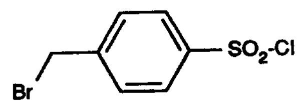

38,1 g (0,2 mol) 4-Methylbenzolsulfonylchlorid werden in 300 ml Tetrachlorkohlenstoff gelöst und mit 35,6 g (0,2 mol) N-Bromsuccinimid versetzt und nach Zugabe von 0,2 g (1,2 mmol) Azobisisobutyronitril (ABU) für 4 h unter Rückfluß erhitzt. Nach dem Abkühlen werden die Feststoffe abfiltriert und das Filtrat vom Lösemittel befreit. Flash-Chromatographie (Petrolether / Toluol 4:1, 50 µm Korngröße) und anschließende Umkristallisation aus 100 ml Cyclohexan ergibt 24,0 g (45% der Theorie) der Titelverbindung.
$R_f$ = 0,75 (Toluol)

Beispiel IV

4-(Brommethyl)-3-chlorbenzolsulfochlorid

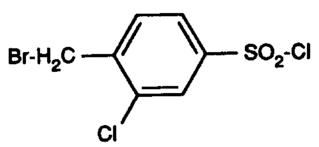

45,9 g (0,2 mol) 3-Chlor-4-methylbenzolsulfonsäure Natriumsalz werden mit 83,3 g (0,4 mol) Phosphorpentachlorid gemischt und 30 min bei 140 ° C Ölbadtemperatur erhitzt. In der Hitze wird mit 500 ml Toluol versetzt, die entstandene Lösung bis zum Sieden erhitzt und nach dem Abkühlen auf Eis gegeben. Die organische Phase wird abgetrennt und mit Wasser gewaschen (2 x 200 ml). Nach dem Trocknen über $MgSO_4$ wird filtriert und alles Flüchtige im Vakuum abgezogen. Der erhaltene Rückstand wird durch Flashchromatographie (Petrolether / Toluol 4:1, 50 µ Korngröße) gereinigt. Man erhält 24,9 g eines Produkts das sofort weiter umgesetzt wird:
Man nimmt in 200 ml Tetrachlorkohlenstoff auf und erhitzt nach Zugabe von 19,6 g (0,11 mol) N-Bromsuccinimid und 0,1 g (0,6 mmol) ABN für 6 h unter Rückfluß. Nach dem Abkühlen werden die Feststoffe abfiltriert und das Filtrat vom Lösemittel befreit. Flashchromatographie (Petrolether / Toluol 4:1, 50 µ Korngröße) ergibt 21,2 g (35%) der Titelverbindung.
$R_f$ = 0,32 (Petrolether / Dichlormethan 4:1)

Beispiel V

4-(Brommethyl)-benzolsulfonyl-N-pyrrolidinid

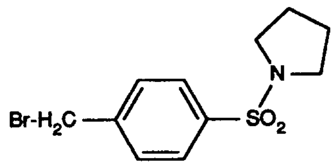

5,3 g (0,02 mol) der Verbindung aus Beispiel III werden in 200 ml Dichlormethan und 4,0 g (0,04 mol) Triethylamin gelöst und nach Zugabe von 1,4 g (0,02 mol) Pyrrolidin in 50 ml Dichlormethan bei 0 °C für 1 h nachgerührt. Man extrahiert mit 2 N HCl (2 x 100 ml), $H_2O$ (2 x 100 ml), trocknet über $MgSO_4$, filtriert und verdampft alle flüchtigen Anteile im Vakuum.
Ausbeute: 5,4 g (89% der Theorie)
$R_f$ = 0,09 (Toluol)

Beispiel VI

4-(Brommethyl)benzolsulfonyl-N-piperidinid

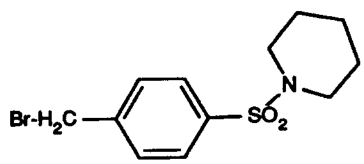

In Analogie zur Vorschrift des Beispiels V erhält man aus 1,1 g (4 mmol) der Verbindung aus Beispiel III und 0,34 g (4 mmol) Piperidin 1,0 g (81% der Theorie) der Titelverbindung.
$R_f$ = 0,14 (Toluol)

Beispiel VII

(S)-4-(Brommethyl)-benzolsulfonyl-N-2-(tert.butoxycarbonyl)pyrrolidinid

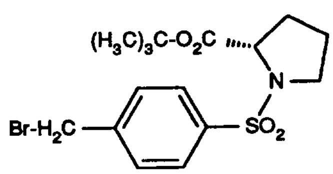

In Analogie zur Vorschrift des Beispiels V erhält man aus 7,25 g (27 mmol) der Verbindung aus Beispiel III und 4,6 g (27 mmol) S-Prolin-tert.butylester 9,1 g (84% der Theorie) der Titelverbindung.
$R_f$ = 0,66 (Petrolether / Essigester 7:3)

Beispiel VIII

rac-4-(Brommethyl)-benzolsulfonyl-N-2-(tert.butoxycarbonyl)piperidinid

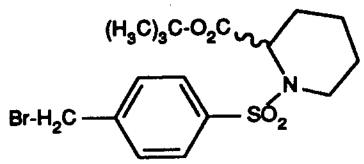

In Analogie zur Vorschrift des Beispiels V erhält man aus 8,0 g (30 mmol) der Verbindung aus Beispiel III und 5,5 g (30 mmol) rac-Pipecolinsäure-tert.butylester 7,4 g (59% der Theorie) der Titelverbindung.
$R_f$ = 0,53 (Petrolether / Essigester 5:1)

Beispiel IX

(S)-4-(Brommethyl)-3-chlorbenzolsulfonyl-N-2-(tert.butoxycarbonyl)pyrrolidinid

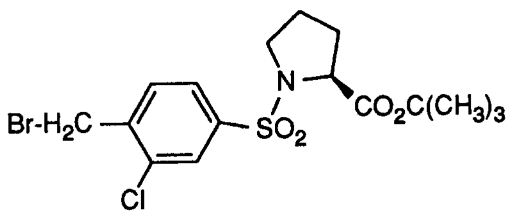

In Analogie zur Vorschrift des Beispiels V erhält man aus 10,0 g (33 mmol) der Verbindung aus Beispiel IV und 5,7 g (33 mmol) S-Prolin-tert.butylester 13,9 g (96% der Theorie) der Titelverbindung.
$R_f$ = 0,55 (Petrolether / Essigester 7:3)

Beispiel X

rac-4-(Brommethyl)-3-chlorbenzolsulfonyl-N-2-(tert.butoxycarbonyl)piperidinid

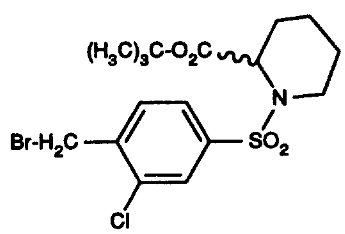

In Analogie zur Vorschrift des Beispiels V erhält man aus 10,0 g (33 mmol) der Verbindung aus Beispiel IV und 6,1 g (33 mmol) rac-Pipecolinsäure-tert.butylester 14,6 g (98% der Theorie) der Titelverbindung.
$R_f$ = 0,6 (Petrolether / Essigester 7:3)

Beispiel XI

6-Butyl-4-benzyloxycarbonyl-2-oxo-1,2-dihydropyridin

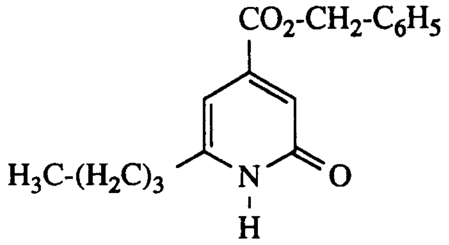

Zu einer Lösung von 6,0 g (31 mmol) 6-Butyl-2-oxo-1,2-dihydro-isonicotinsäure in 100 ml DMF gibt man 13,3 g (123 mmol) Benzylalkohol und 4,7 g (31 mmol) Hydroxybenzotriazol. Die entstandene Klare Lösung wird auf 0 ° C gekühlt, gefolgt von der Zugabe von 7,0 g (34 mmol) Dicyclohexylcarbodiimid und 4,2 ml (31 mmol) Triethylamin. Man läßt auf 20 ° C auftauen, rührt noch 2 Stunden nach und arbeitet wäßrig auf. Man erhält 6,8 g (77% der Theorie) der Titelverbindung.
Fp.: 139 ° C

Beispiel XII

4-Methoxycarbonyl-2-oxo-6-propyl-1,2-dihydropyridin

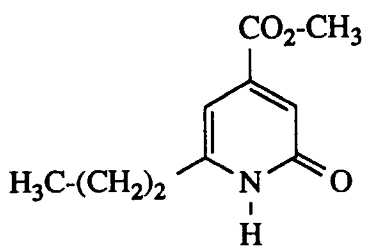

In Analogie zur Vorschrift des Beispiels I erhält man aus 14,5 g (80 mmol) 2-Oxo-6-propyl-1,2-dihydro-isonicotinsäure und Methanol 13,7 g (88 % d.Th.) die Titelverbindung.
F ° C: 144

Beispiel XIII

N-Trifluoracetyl-L-prolinamid

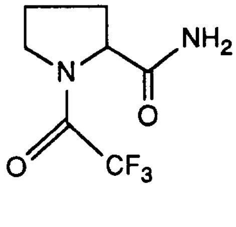

30 g (0,142 mol) Trifluoracetylprolin werden unter Schutzgas in 150 ml DMF vorgelegt. Bei -20 ° C gibt man 142,6 ml (0,1704 mol) 38 %iges PPA in Essigester zu. Es wird bis zur Sättigung Ammoniak eingeleitet, wobei nach 30 min ein weißer Niederschlag ausfällt Unter schwachem Ammoniakstrom wird der Ansatz

aufgetaut. Dann gibt man die gesamte Reaktionsmischung in 600 ml $H_2O$ und säuert mit konzentrierter Essigsäure bis pH 4 an. Es wird 4 x mit 200 ml Methylenchlorid und 3 x mit 200 ml Ether ausgeschüttelt. Die vereinigten organischen Phasen werden mit Magnesiumsulfat getrocknet und das Lösungsmittel abgezogen. Die Rückstände chromatographiert man zusammen über Kieselgel 60 F254 Methylenchlorid/Methanol (10:1). Die das Produkt enthaltenen Fraktionen werden am Rotationsverdampfer vom Lösungsmittel befreit.
Man erhält 17,12 g der Titelverbindung (57 % der Theorie);
$R_f$: 0,345 (T/EE/$CH_3COOH$) 20:20:1.

Beispiel XIV

2-Cyano-N-trifluoracetyl-pyrrolidin

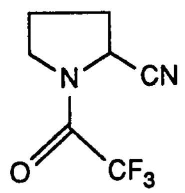

40 g (0,19 mol) des Produkts aus Beispiel XIII und 45 g = 46 ml (0,57 mol) Pyridin legt man unter Schutzgas in 300 ml THF vor. Bei 0 °C tropft man 48 g = 32,25 ml (0,228 mol) Trifluoressigsäureanhydrid zu. Die Reaktionsmischung wird 30 min bei 0 °C und 90 min bei Raumtemperatur nachgerührt. Der Ansatz wird dann in 1 l 1N Salzsäure gegeben und 3 x mit 200 ml Methylenchlorid ausgeschüttelt. Die vereinigten organischen Phasen werden mit 200 ml gesättigter NaCl-Lösung ausgeschüttelt und über Magnesiumsulfat getrocknet. Das Lösungsmittel wird abgezogen und der Rückstand wird an Kieselgel 60 F254 chromatographiert.
Petrolether/Essigester/Essigsäure (1600:200:5). Die das Produkt enthaltenen Fraktionen engt man ein. Man erhält 32,4 g der Titelverbindung (88,8 % der Theorie).
$R_f$: 0,57 (PE/EE 7:3).

Beispiel XV

2-Tetrazolyl-N-trifluoracetyl-pyrrolidin

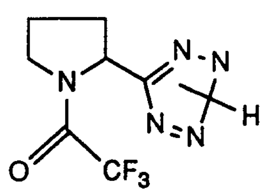

31,35 g = 32,6 ml (0,26 mol) Diethylaluminiumchlorid werden in 65 ml Toluol unter Schutzgas vorgelegt. Bei Raumtemperatur gibt man 29,95 g = 34,04 ml (0,26 mol) Trimethylsilylazid zu und es wird 10 min bei Raumtemperatur nachgerührt. Bei 0 °C gibt man 25 g (0,13 mol) des Produkts aus Beispiel XIV, gelöst in 65 ml Toluol, hinzu. Die Reaktionsmischung wird 30 min bei 0 °C, 120 min bei Raumtemperatur und 60 min bei 40 °C gerührt. Der abgekühlte Ansatz wird so lange mit gesättigter Kaliumfluorid-Lösung versetzt, bis keine Gasentwicklung mehr zu erkennen ist.

Die Reaktionsmischung wird in 600 ml $H_2O$ gegeben und bis pH 4 angesäuert und 3 x mit 100 ml Essigester extrahiert. Die vereinigten organischen Phasen werden mit 50 ml n-Hexan versetzt. Um die Azide zu entfernen, wird ca. 1/3 des Lösungsmittels über eine Destillationsbrücke ohne Kühlung abdestilliert. Der Rückstand wird über Magnesiumsulfat getrocknet und am Rotationsverdampfer vom Lösungsmittel befreit.
Man erhält 18,54 g der Titelverbindung (60,6 % der Theorie).
$R_f$: 0,4 (Toluol/Essigester 1:1).

Beispiel XVI

N-Trifluoracetyl-2-[N-trityl-tetrazolyl]pyrrolidin

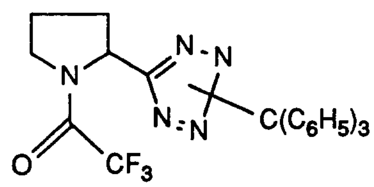

16,23 g (0,069 mol) des Produktes aus Beispiel XV und 10,47 g = 14,35 ml (0,1035 mol) Triethylamin werden in 70 ml Methylenchlorid vorgelegt. Dann gibt man 19,83 g (0,069 mol) Triphenylmethylchlorid zu. Man läßt die Reaktionsmischung 1,5 h bei Raumtemperatur nachrühren, verdünnt mit Methylenchlorid und extrahiert mit pH = 5 Pufferlösung (3 x 50 ml). Die organische Phase wird über Magnesiumsulfat getrocknet. Das Lösungsmittel wird am Rotationsverdampfer abgezogen. Der Rückstand wird mit Ether verrührt. Die entstandenen Kristalle werden abgesaugt und getrocknet.
Man erhält 24,65 g der Titelverbindung (75 % der Theorie).
$R_f$: 0,53 (Petrolether/Essigester 7:3).

Beispiel XVII

2-(N-Trityl-tetrazolyl)pyrrolidin

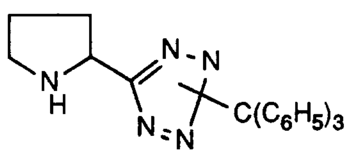

24 g (0,05 mol) des Produktes aus Beispiel XVI werden unter Schutzgas in 100 ml Ethanol vorgelegt. Bei 0 °C gibt man portionsweise 2,84 g (0,075 mol) Natriumborhydrid zu. Der Ansatz wird aufgetaut und 1 h bei Raumtemperatur gerührt. Man versetzt mit 6 ml Essigsäure und gibt die ganze Reaktionsmischung in 500 ml Pufferlösung pH 9. Der Ansatz wird mit 3 x 75 ml Methylenchlorid extrahiert. Die vereinigten organischen Phasen werden über Magnesiumsulfat getrocknet und am Rotationsverdampfer vom Lösungsmittel befreit. Der Rückstand wird an Kieselgel 60 F254 chromatographiert. Petrolether/Essigester (7:3). Die entsprechenden Fraktionen werden eingeengt und getrocknet.
Man erhält 7,16 g der Titelverbindung (37,5 % der Theorie).
$R_f$: 0,22 (Essigester).

Beispiel XVIII

4-Brommethyl-3-chlor-benzolsulfonsäure-2-[trityl-tetrazolyl]pyrrolidinid

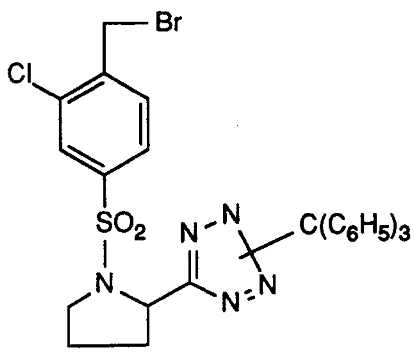

In Analogie zur Vorschrift des Beispiels III erhält man aus 3,19 g (10,5 mmol) der Verbindung aus Beispiel IV und 4 g (10,5 mmol) der Verbindung aus Beispiel XVII 6,49 g der Titelverbindung (95 % der Theorie).
$R_f$: 0,53 (Petrolether/Essigester 7:3).

Beispiel XIX

4-(Brommethyl)-3-fluorbenzolsulfochlorid

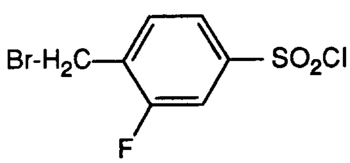

Man nimmt 20,9 g (0,1 mol) 3-Fluor-4-methylbenzolsulfochlorid in 200 ml Tetrachlorkohlenstoff auf und erhitzt nach Zugabe von 19,6 g (0,11 mol) N-Bromsuccinimid und 0,3 g Dibenzoylperoxid für 5 h unter Rückfluß. Nach dem Abkühlen werden die Feststoffe abfiltriert und das Filtrat vom Lösemittel befreit. Flashchromatographie Petrolether/Toluol (4:1), 50 µm Korngröße ergibt 12,4 g (44 % der Theorie) der Titelverbindung.
$R_f$: 0,42 (Petrolether/Toluol 3:1).

Beispiel XX

4-(Brommethyl)-3-trifluormethylbenzolsulfochlorid

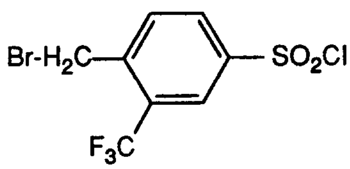

Man nimmt 64,6 g (0,25 mol) 3-Trifluormethyl-4-methylbenzolsulfochlorid in 500 ml Tetrachlorkohlenstoff auf und erhitzt nach Zugabe von 44,5 g (0,25 mol) N-Bromsuccinimid und 0,4 g ABN für 24 h unter

Rückfluß. Nach dem Abkühlen werden die Feststoffe abfiltriert und das Filtrat vom Lösemittel befreit. Flashchromatographie Petrolether/Toluol (4:1), 50 µm Korngröße ergibt 33,9 g (40 % der Theorie) der Titelverbindung.
$R_f$: 0,41 (Petrolether/Toluol 3:1).

Beispiel XXI

(S)-4-(Brommethyl)-3-fluorbenzolsulfonyl-N-2-(tert.butoxy-carbonyl)pyrrolidinid

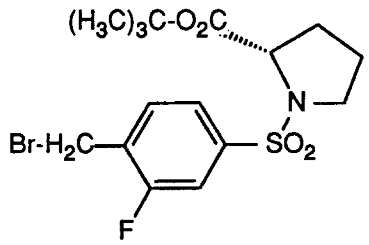

In Analogie zur Vorschrift aus Beispiel III erhält man aus 8,6 g (30 mmol) der Verbindung aus Beispiel XIX und 5,1 g (30 mmol) S-Prolin-tert.-butylester 12,7 g (100 % der Theorie) der Titelverbindung.
$R_f$: 0,57 (Petrolether/Essigester 7:3).

Beispiel XXII

(S)-4-(Brommethyl)-3-trifluormethylbenzolsulfonyl-N-2-(tert.-butoxycarbonyl)-pyrrolidinid

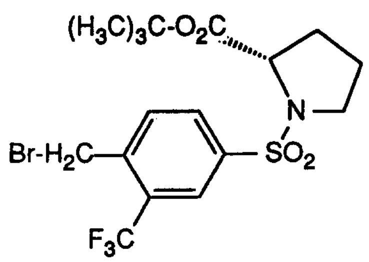

In Analogie zur Vorschrift des Beispiels III erhält man aus 16,9 g (50 mmol) der Verbindung aus Beispiel XX und 8,6 g (50 mmol) S-Prolin-tert.-butylester 23,6 g (100 % der Theorie) der Titelverbindung.
$R_f$: 0,63 (Petrolether/Essigester 7:3).

Beispiel XXIII

(S)-4-Carboxy-3-hydroxybenzolsulfonyl-N-2(tert.-butoxycarbonyl)pyrrolidinid

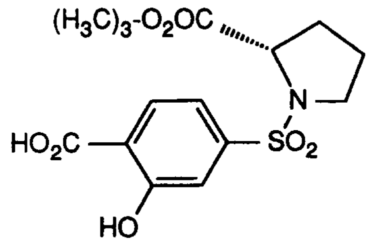

In Analogie zur Vorschrift des Beispiels III erhält man aus 23,7 g 4-Carboxy-3-hydroxybenzolsulfochlorid (100 mmol) und 17,1 g (100 mmol) S-Prolin-tert.-butylester 30,0 g (81 % der Theorie) der Titelverbindung. $R_f$: 0,18 (Aceton)

Beispiel XXIV

(S)-4-Benzoyloxycarbonyl-3-benzyloxybenzolsulfonsäure-N-2-(tert.-butoxycarbonyl)pyrrolidinid

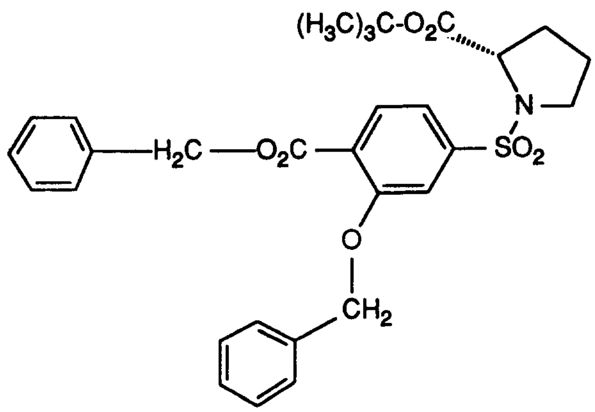

25,3 g (68 mmol) der Verbindung aus Beispiel XXIII gelöst in 200 ml DMF werden mit 28,3 g $K_2CO_3$ - (204 mmol) und 25,7 g (150 mmol) Benzylbromid versetzt. Man läßt die Reaktionsmischung 2 Stunden bei 75 °C nachrühren, gibt nach dem Abkühlen 1 l Wasser hinzu, extrahiert mit Essigester (3 x 400 ml), wäscht mit Wasser (5 x 400 ml), trocknet über $MgSO_4$, filtriert und zieht alles Flüchtige im Vakuum ab. Man reinigt durch Flash-Chromatographie (Petrolether/$CH_2Cl_2$ 5:1 und Petrolether/Essigester 6:1, 50 µm Teilchengröße). Zur weiteren Reinigung wird das Produkt aus 600 ml eines Lösungsmittelgemisches (Petrolether/Essigester 6:1) umkristallisiert und man erhält 35,5 g (95 % der Theorie) der Titelverbindung. $R_f$ = 0,53 (Petrolether/Essigester 7:3).

Beispiel XXV

(S)-4-(Hydroxymethyl)-3-benzyloxybenzolsulfonsäure-N-2-(tert.-butoxycarbonyl)-pyrrolidinid

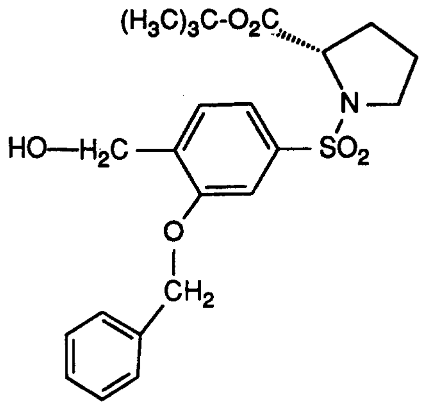

11,03 g (20 mmol) der Verbindung aus Beispiel XXIV werden in 100 ml Diglycene gelöst und nach Zugabe von 1,51 g (40 mmol) Natriumborhydrid und 1,68 g (40 mmol) LiCl für 4 Stunden bei 70 °C nachgerührt. Nach dem Abkühlen wird das Reaktionsgemisch mit 500 ml Wasser versetzt und mit 1 H HCl bis pH 3 angesäuert. Es wird mit Ether extrahiert (3 x 300 ml), mit Wasser gewaschen (6 x 300 ml), über $MgSO_4$ getrocknet und das Filtrat vom Lösemittel befreit. Der Rückstand wird an Kieselgel 60 F 254 chromatographiert. Petrolether/Essigester (7:3). Die entsprechenden Fraktionen werden eingeengt und getrocknet. Man erhält 5,0 g (56 % der Theorie) der Titelverbindung.
$R_f$: 0,36 (Petrolether/Essigester 7:3).

Beispiel XXVI

(S)-4-(Brommethyl)-3-benzyloxybenzolsulfonsäure-N-2-(tert.butoxycarbonyl)-pyrrolidinid

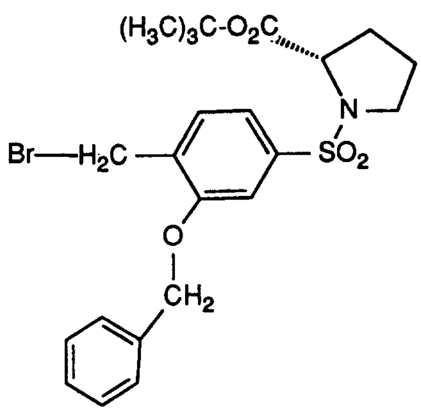

2,24 g (5 mmol) der Verbindung aus Beispiel XXV werden unter Schutzgas in 20 ml absolutem DMF vorgelegt. Bei 0 °C gibt man 2,53 g (6 mmol) Triphenylphosphindibromid hinzu. Das Reaktionsgemisch wird 1 Stunde bei Raumtemperatur gerührt. Man versetzt mit 200 ml Wasser, extrahiert mit Essigester (3 x 80 ml), wäscht mit Wasser (5 x 60 ml), trocknet über $MgSO_4$, filtriert und zieht alles Flüchtige im Vakuum ab. Man reinigt durch Flash-Chromatogaphie ($CH_2Cl_2$, 50 $\mu$m Teilchengröße) und erhält 2,55 g (100 % der Theorie) der Titelverbindung.
$R_f$: 0,56 (Petrolether/Essigester 7:3).

Herstellungsbeispiele

Beispiel 1

rac-4-[4-Benzyloxycarbonyl-6-butyl-2-oxo-1,2-dihydropyridin-1-yl]-methyl-3-chlor-benzolsulfonyl-N-(2-tert.butoxycarbonyl)piperidinid

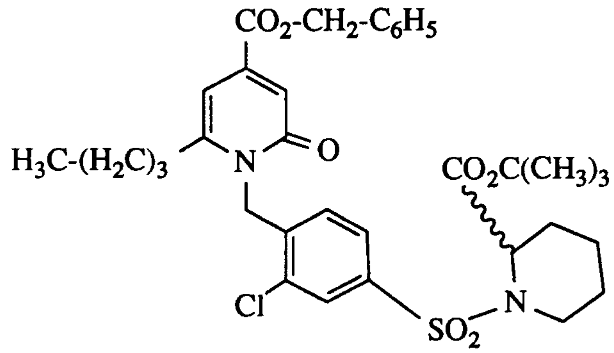

500 mg (1,75 mmol) des Produkts aus Beispiel X und 570 mg (1,75 mmol) $Cs_2CO_3$ werden in 10 ml trockenem 1,2-Dimethoxyethan suspendiert und 10 min bei 20 °C gerührt. Man gibt 790 mg (1,75 mmol) des Produkts aus Beispiel VII, gelöst in 10 ml 1,2-Dimethoxyethan zu und rührt 5 h bei 20 °C nach. Man kühlt ab, gibt die Reaktionsmischung zu 100 ml $H_2O$, extrahiert mit Ethylacetat (4 x 60 ml), trocknet über $MgSO_4$ und zieht das Lösemittel im Vakuum ab. Das Rohprodukt wird an Kieselgel (50 $\mu$ Teilchengröße, Laufmittel Petrolether / Ethylacetat 10:1 -> 7:3) chromatographiert. Man erhält 78 mg (8% der Theorie) der Titelverbindung.
$R_f$: 0,09 (Petrolether / Ethylacetat 5:1)

Beispiel 2

rac-4-[4-benzyloxycarbonyl-6-butyl-2-oxo-1,2-dihydropyridin-1-yl]methyl-3-chlor-benzolsulfonyl-N-(2-carboxy)piperidinid

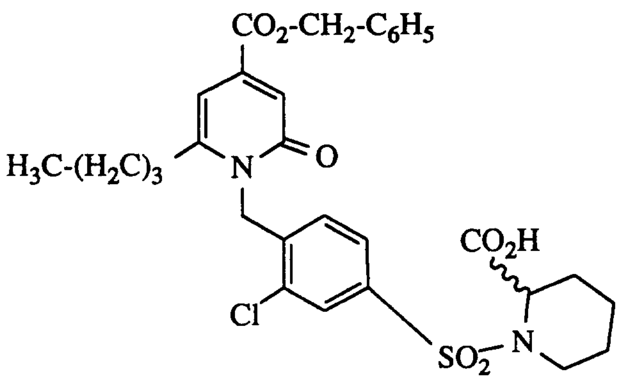

Zu einer Lösung von 76 mg (126 $\mu$mol) der Verbindung aus Beispiel 1 in 2 ml Dichlormethan gibt man 400 ml Trifluoressigsäure und rührt 5 h bei 20 °C. Man verdünnt mit 10 ml Dichlormethan, wäscht mit Wasser (4 x 20 ml) und gesättigter NaCl-Lösung (1 x 20 ml), trocknet über $MgSO_4$ und zieht das Lösemittel im Vakuum ab. Der Rückstand wird durch Flash-Chromatographie (50 $\mu$ Teilchengröße, Laufmittel: Toluol / Ethylacetat / Trifluoressigsäure 10:40:1) gereinigt an 20 g Kieselgel. Man erhält 63 mg (83% der Theorie) der Titelverbindung.

$R_f$ = 0,66 (Dichlormethan / Methanol 10:1)

Beispiel 3

rac-4-[6-Butyl-4-carboxy-2-oxo-1,2-dihydropyridin-1-yl]methyl-3-chlor-benzolsulfonyl-N-(2-carboxy)-piperidinid

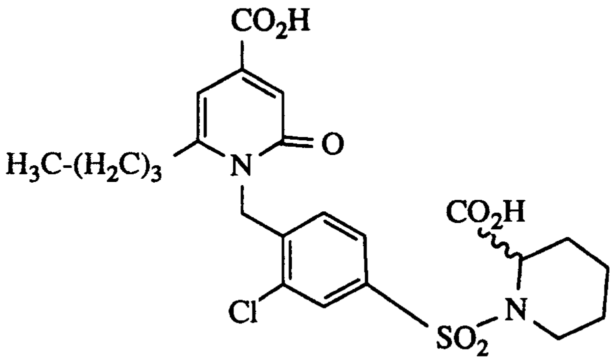

58 mg (96 µmol) der Verbindung aus Beispiel 1 werden in 0,5 ml THF, 0,5 ml $H_2O$ und 0,1 ml $CH_3OH$ gelöst. Nach Zugabe von 12 mg (129 µmol) LiOH wird 2,5 h bei RT gerührt. Die Lösung wird eingeengt, mit $H_2O$ und Ethylacetat versetzt und mit Essigsäure bis auf pH = 3 angesäuert. Die Phasen werden getrennt und die wäßrige Phase noch dreimal mit Ethylacetat extrahiert. Die vereinigten organischen Phasen werden mit Wasser (7 x 20 ml) und gesättiger NaCl-Lösung (1 x 20 ml) gewaschen, über $MgSO_4$ getrocknet und eingeengt. Man erhält 36 mg (74%) der Titelverbindung.
$R_f$ = 0,16 (Dichlormethan/Methanol 10:1).

In Analogie zu den Vorschriften der Herstellungsbeispiele 1 - 3 werden die in Tabelle 1 aufgeführten Verbindungen hergestellt:

Tabelle 1:

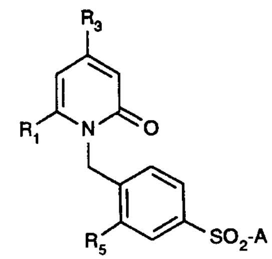

| Bsp.-Nr. | $R^1$ | $R^3$ | $R^5$ | A | $R_f$* |
|---|---|---|---|---|---|
| 4 | $-(CH_2)_3-CH_3$ | $-CO_2CH_2C_6H_5$ | Cl | 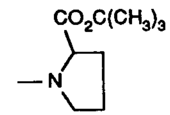 | 0,9 [a] |
| 5 | $-(CH_2)_3-CH_3$ | $-CO_2CH_2C_6H_5$ | Cl | 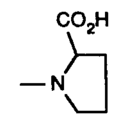 | 0,52 [a] |
| 6 | $-(CH_2)_3-CH_3$ | $-CO_2H$ | Cl | 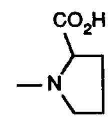 | 0,14 [a] |
| 7 | $-(CH_2)_3-CH_3$ | $-CO_2CH_3$ | H | 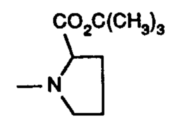 | |
| 8 | $-(CH_2)_3-CH_3$ | $-CO_2CH_3$ | H | 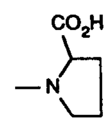 | |
| 9 | $-(CH_2)_3-CH_3$ | $-CO_2H$ | H | 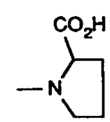 | |

<u>Fortsetzung Tabelle 1:</u>

| Bsp.-Nr. | $R^1$ | $R^3$ | $R^5$ | A | $R_f$* |
|---|---|---|---|---|---|
| 10 | $--(CH_2)_2-CH_3$ | $-CO_2CH_3$ | H | 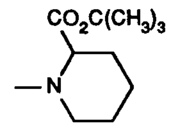 | 0,35[a] |
| 11 | $-(CH_2)_2-CH_3$ | $-CO_2CH_3$ | H | 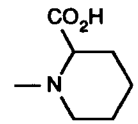 | 0,36[a] |
| 12 | $-(CH_2)_3-CH_3$ | $-CO_2CH_3$ | H | 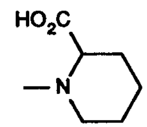 | 0,42[a)] |
| 13 | $-(CH_2)_3-CH_3$ | $-CO_2H$ | H | 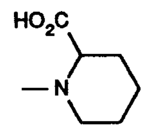 | 0,34[b)] |
| 14 | $-(CH_2)_3-CH_3$ | $-CO_2Me$ | Cl | 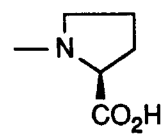 | 0,60[a)] |

Beispiel 15

rac-4-[6-Butyl-4-carboxy-2-oxo-1,2-dihydropyridin-1-yl]-4-methyl-benzolsulfonyl-N-(2-carboxy)piperidinid-di-Natrium-Salz

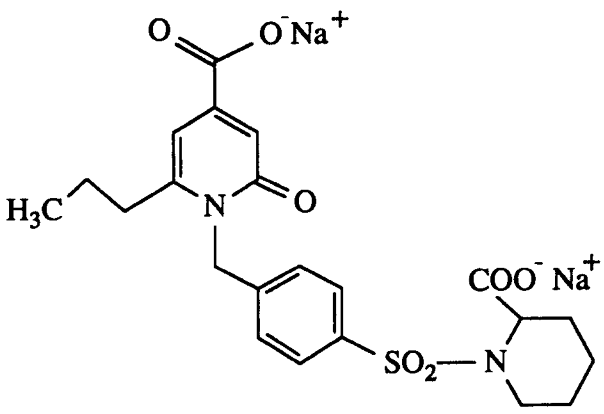

120 mg (0,25 mmol) der Verbindung aus Beispiel 11 werden in 5 ml THF und 2,5 ml Wasser mit 0,25 ml 1 N Natronlauge 1 Stunde bei Raumtemperatur versetzt, die Reaktionslösung eingeengt und über $P_2O_5$ im Hochvakuum getrocknet.
Ausbeute: 115 mg (91% der Theorie)
$R_f$ = 0,28[b)]
In Analogie zur Vorschrift des Beispiels 14 werden die in Tabelle 2 aufgeführten Verbindungen durch Einsatz von 1 Mol-Äquivalent NaOH hergestellt.

Tabelle 2:

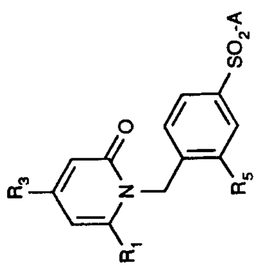

| Bsp.-Nr. | $R^1$ | $R^3$ | $R^5$ | A | $R_f$* |
|---|---|---|---|---|---|
| 16 | $-(CH_2)_2-CH_3$ | $-CO_2CH_3$ | H | 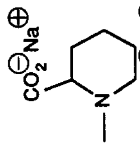 | 0,36[a)] |
| 17 | $-(CH_2)_3-CH_3$ | $-CO_2CH_3$ | H | 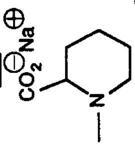 | 0,42 [a)] |
| 18 | $-(CH_2)_3-CH_3$ | $-CO_2CH_3$ | H | 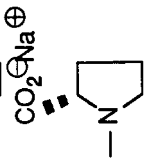 | 0,22[c)] |
| 19 | $-(CH_2)_3-CH_3$ | $-CO_2Me$ | Cl | 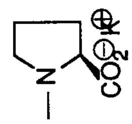 | 0,60[a)] |
| 20 | $-(CH_2)_3-CH_3$ | $-CO_2H$ | Cl | 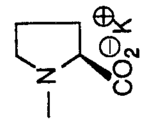 | |

## Patentansprüche

**1.** Sulfonylbenzyl-substituierte Pyridone der allgemeinen Formel

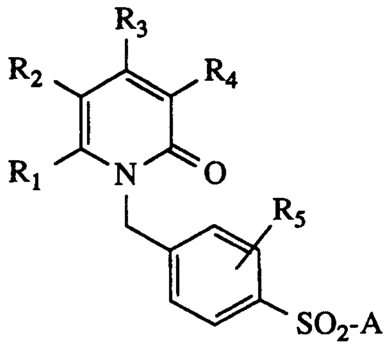

(I),

in welcher

$R^1$ für geradkettiges oder verzweigtes Alkyl mit bis zu 8 Kohlenstoffatomen steht, das gegebenenfalls durch Cycloalkyl mit 3 bis 6 Kohlenstoffatomen, Hydroxy oder durch geradkettiges oder verzweigtes Alkoxy mit bis zu 6 Kohlenstoffatomen substituiert ist, oder für Cycloalkyl mit 3 bis 6 Kohlenstoffatomen steht,

$R^2$, $R^3$ und $R^4$ gleich oder verschieden sind und für Wasserstoff, Cyano oder geradkettiges oder verzweigtes Perfluoralkyl mit bis zu 8 Kohlenstoffatomen stehen, oder

für geradkettiges oder verzweigtes Alkyl mit bis zu 8 Kohlenstoffatomen stehen, das gegebenenfalls bis zu 2-fach gleich oder verschieden durch Hydroxy, Halogen, Carboxy, geradkettiges oder verzweigtes Alkoxy oder Alkoxycarbonyl mit jeweils bis zu 6 Kohlenstoffatomen, oder durch Phenyl, Phenoxy oder durch einen 5- bis 7-gliedrigen, gesättigten oder ungesättigten Heterocyclus mit bis zu 3 Heteroatomen substituiert ist, wobei die Cyclen ihrerseits bis zu 2-fach gleich oder verschieden durch Trifluormethyl, Trifluormethoxy, Halogen, Nitro, Cyano, Hydroxy, Hydroxymethyl oder

durch geradkettiges oder verzweigtes Alkyl oder Alkoxy mit jeweils bis zu 6 Kohlenstoffatomen substituiert sein können, oder

für geradkettiges oder verzweigtes Acyl oder Alkoxycarbonyl mit jeweils bis zu 8 Kohlenstoffatomen, Benzyloxycarbonyl oder Carboxy stehen, oder

für Phenyl stehen, das gegebenenfalls bis zu 3-fach gleich oder verschieden durch Halogen, Nitro, Cyano, Hydroxy, Hydroxymethyl, Trifluormethyl, Trifluormethoxy oder durch geradkettiges oder verzweigtes Alkyl oder Alkoxy mit jeweils bis zu 6 Kohlenstoffatomen substituiert ist, oder

für eine Gruppe der Formel $-CO-NR^6R^7$ stehen,

worin

$R^6$ und $R^7$ gleich oder verschieden sind und Wasserstoff, geradkettiges oder verzweigtes Alkyl mit bis zu 8 Kohlenstoffatomen oder Aryl oder Aralkyl mit 6 bis 10 Kohlenstoffatomen bedeuten,

$R^5$ für Wasserstoff, Halogen oder geradkettiges oder verzweigtes Alkyl mit bis zu 8 Kohlenstoffatomen, oder

für geradkettiges oder verzweigtes Perfluoralkyl mit bis zu 6 Kohlenstoffatomen steht,

oder

für eine Gruppe der Formel -OX steht,

worin

X Wasserstoff, Benzyl, eine Hydroxyschutzgruppe oder geradkettiges oder verzweigtes Alkyl mit bis zu 8 Kohlenstoffatomen bedeutet,

A für einen über das Stickstoffatom gebundenen 3 bis 8-gliedrigen, gesättigten Heterocyclus mit bis zu 2 weiteren Heteroatomen aus der Reihe S, N oder O steht und der gegebenenfalls bis zu 2-fach gleich oder verschieden durch Perfluoralkyl mit bis zu 5 Kohlenstoffatomen oder durch einen Rest der Formel

$-SO_3H$ ,

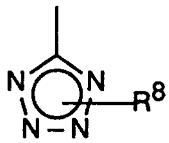 ,

$-CO-R^9$ oder

$$\overset{\displaystyle O}{\overset{\|}{-P}}(OR^{10})(OR^{11})$$

substituiert ist,
worin

$R^8$ Wasserstoff, geradkettiges oder verzweigtes Alkyl mit bis zu 6 Kohlenstoffatomen oder Triphenylmethyl bedeutet,

$R^9$ Hydroxy, geradkettiges oder verzweigtes Alkoxy mit bis zu 8 Kohlenstoffatomen, Phenoxy, Benzyloxy oder eine Gruppe der Formel $-NR^{12}R^{13}$ bedeutet,
worin

$R^{12}$ und $R^{13}$ gleich oder verschieden sind und Wasserstoff oder geradkettiges oder verzweigtes Alkyl mit bis zu 6 Kohlenstoffatomen oder Phenyl bedeuten,

$R^{10}$ und $R^{11}$ gleich oder verschieden sind und Wasserstoff, geradkettiges oder verzweigtes Alkyl mit bis zu 8 Kohlenstoffatomen oder Phenyl bedeuten,

und deren Salze.

**2.** Sulfonylbenzyl-substituierte Pyridone nach Anspruch 1,
wobei

$R^1$ für geradkettiges oder verzweigtes Alkyl mit jeweils bis zu 6 Kohlenstoffatomen steht, das gegebenenfalls durch Cyclopropyl, Cyclopentyl, Cyclohexyl, Hydroxy oder durch geradkettiges oder verzweigtes Alkoxy mit bis zu 4 Kohlenstoffatomen substituiert ist, oder für Cyclopropyl, Cyclopentyl oder Cyclohexyl steht,

$R^2$, $R^3$ und $R^4$ gleich oder verschieden sind und für Wasserstoff, Cyano oder für geradkettiges oder verzweigtes Perfluoralkyl mit bis zu 6 Kohlenstoffatomen stehen, oder
für geradkettiges oder verzweigtes Alkyl mit bis zu 6 Kohlenstoffatomen stehen, das durch Hydroxy, Fluor, Chlor, Brom, Carboxy, geradkettiges oder verzweigtes Alkoxy oder Alkoxycarbonyl mit jeweils bis zu 4 Kohlenstoffatomen, oder durch Phenyl, Phenoxy oder Thienyl substituiert ist, wobei die Cyclen ihrerseits durch Trifluormethoxy, Trifluormethyl, Hydroxymethyl, Fluor, Chlor, Brom oder
durch geradkettiges oder verzweigtes Alkyl oder Alkoxy mit jeweils bis zu 6 Kohlenstoffatomen substituiert sein können, oder
für geradkettiges oder verzweigtes Acyl oder Alkoxycarbonyl mit jeweils bis zu 6 Kohlenstoffatomen, Benzyloxycarbonyl oder Carboxy stehen, oder
für Phenyl stehen, das gegebenenfalls bis zu 2-fach gleich oder verschieden durch Fluor, Chlor, Brom, Trifluormethyl, Trifluormethoxy, Hydroxymethyl oder durch geradkettiges oder verzweigtes Alkyl oder Alkoxy mit jeweils bis zu 4 Kohlenstoffatomen substituiert ist, oder
für eine Gruppe der Formel $-CONR^6R^7$ stehen,
worin

$R^6$ und $R^7$ gleich oder verschieden sind und Wasserstoff, geradkettiges oder verzweigtes Alkyl mit bis zu 6 Kohlenstoffatomen, Phenyl oder Benzyl bedeuten,

$R^5$ für Wasserstoff, Fluor, Chlor, Brom, geradkettiges oder verzweigtes Alkyl mit bis zu 6 Kohlenstoffatomen, oder
für geradkettiges oder verzweigtes Perfluoralkyl mit bis zu 4 Kohlenstoffatomen steht,
oder

für eine Gruppe der Formel -OX steht,
worin

X — Wasserstoff, Benzyl, Acetyl oder geradkettiges oder verzweigtes Alkyl mit bis zu 6 Kohlenstoffatomen bedeutet,

A — für über das Stickstoffatom gebundenes Piperidyl, Pyrrolidinyl oder Morpholinyl steht, die gegebenenfalls durch Trifluormethyl oder durch einen Rest der Formel $-SO_3H$ ,

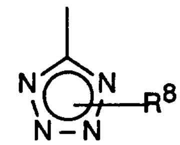 ,

$-CO-R^9$ oder

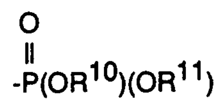

substituiert sind,
worin

$R^8$ — Wasserstoff, geradkettiges oder verzweigtes Alkyl mit bis zu 4 Kohlenstoffatomen oder Triphenylmethyl bedeutet,

$R^9$ — Hydroxy, geradkettiges oder verzweigtes Alkoxy mit bis zu 6 Kohlenstoffatomen, Phenoxy, Benzyloxy oder eine Gruppe der Formel $-NR^{12}R^{13}$ bedeutet,
worin

$R^{12}$ und $R^{13}$ — gleich oder verschieden sind und Wasserstoff oder geradkettiges oder verzweigtes Alkyl mit bis zu 4 Kohlenstoffatomen bedeuten,
und

$R^{10}$ und $R^{11}$ — gleich oder verschieden sind und Wasserstoff, geradkettiges oder verzweigtes Alkyl mit bis zu 6 Kohlenstoffatomen oder Phenyl bedeuten.

und deren Salze.

**3.** Sulfonylbenzyl-substituierte Pyridone nach Anspruch 1,
wobei

$R^1$ — für geradkettiges oder verzweigtes Alkyl mit bis zu 4 Kohlenstoffatomen steht, das gegebenenfalls durch Cyclopropyl substituiert ist oder
für Cyclopropyl steht,

$R^2$, $R^3$ und $R^4$ — gleich oder verschieden sind und für Wasserstoff, Cyano oder für geradkettiges oder verzweigtes Perfluoralkyl mit bis zu 4 Kohlenstoffatomen stehen,
für geradkettiges oder verzweigtes Alkyl mit bis zu 4 Kohlenstoffatomen stehen, das durch Hydroxy oder geradkettiges oder verzweigtes Alkoxy oder Alkoxycarbonyl mit jeweils bis zu 3 Kohlenstoffatomen substituiert ist, oder
für geradkettiges oder verzweigtes Acyl oder Alkoxycarbonyl mit jeweils bis zu 4 Kohlenstoffatomen, Benzyloxycarbonyl oder Carboxy stehen, oder
für eine Gruppe der Formel $-CONR^6R^7$ stehen,
worin

$R^6$ und $R^7$ — gleich oder verschieden sind und Wasserstoff, geradkettiges oder verzweigtes Alkyl mit bis zu 4 Kohlenstoffatomen, Phenyl oder Benzyl bedeuten,

$R^5$ — für Wasserstoff, Fluor, Chlor, geradkettiges oder verzweigtes Alkyl mit bis zu 4 Kohlenstoffatomen, oder
für geradkettiges oder verzweigtes Perfluoralkyl mit bis zu 3 Kohlenstoffatomen steht,
oder
für eine Gruppe der Formel -OX steht,

worin

X Wasserstoff, Benzyl, Acetyl oder geradkettiges oder verzweigtes Alkyl mit bis zu 6 Kohlenstoffatomen bedeutet,

A für über das Stickstoffatom gebundenes Piperidyl oder Pyrrolidinyl steht, die gegebenenfalls durch Trifluormethyl oder durch einen Rest der Formel

$-SO_3H$ ,

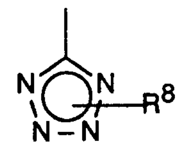 ,

$-CO-R^9$ oder

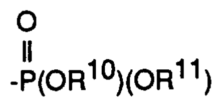

substituiert sind,

worin

$R^8$ Wasserstoff, Methyl, Ethyl oder Triphenylmethyl bedeutet

und

$R^9$ Hydroxy, geradkettiges oder verzweigtes Alkoxy mit bis zu 4 Kohlenstoffatomen, Phenoxy, Benzyloxy oder eine Gruppe der Formel $-NR^{11}R^{13}$ bedeutet,

worin

$R^{12}$ und $R^{13}$ gleich oder verschieden sind und Wasserstoff oder geradkettiges oder verzweigtes Alkyl mit bis zu 3 Kohlenstoffatomen bedeuten

$R^{10}$ und $R^{11}$ gleich oder verschieden sind und Wasserstoff, geradkettiges oder verzweigtes Alkyl mit bis zu 4 Kohlenstoffatomen oder Phenyl bedeuten,

und deren Salze.

**4.** Sulfonylbenzyl-substituierte Pyridone nach Anspruch 1 zur therapeutischen Anwendung.

**5.** Verfahren zur Herstellung von Sulfonylbenzyl-substituierten Pyridonen nach Anspruch 1 dadurch gekennzeichnet, daß man Pyridone der allgemeinen Formel (II)

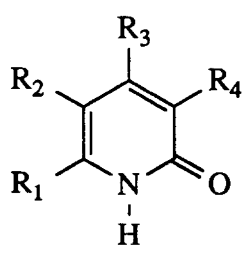 (II),

in welcher

$R^1$, $R^2$, $R^3$ und $R^4$ die oben angegebene Bedeutung haben,

mit Verbindungen der allgemeinen Formel (III)

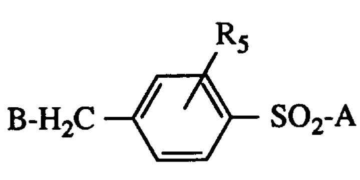 (III),

in welcher

| | |
|---|---|
| $R^5$ und A | die oben angegebene Bedeutung haben und |
| B | für Halogen, vorzugsweise für Brom steht, |

in organischen Lösemitteln und in Anwesenheit einer Base und gegebenenfalls eines Katalysators umsetzt,
und im Fall, daß $R^8 \neq$ Wasserstoff eine Alkylierung anschließt und im Fall der Säuren ($R^9$ = OH) die entsprechenden Ester verseift
und im Fall der Ester oder Amide, gegebenenfalls über eine aktivierte Carbonsäurestufe, eine Veresterung bzw. Amidierung anschließt
und die Substituenten $R^2$, $R^3$, $R^4$ und $R^5$ nach üblichen Methoden variiert.

**6.** Verfahren nach Anspruch 5 dadurch gekennzeichnet, daß man die Umsetzung in einen Temperaturbereich von -100 °C bis +100 °C durchführt.

**7.** Arzneimittel enthaltend mindestens ein Sulfonylbenzyl-substituiertes Pyridon nach Anspruch 1.

**8.** Arzneimittel nach Anspruch 7 zur Behandlung von arterieller Hypertonie und Atherosklerose.

**9.** Verwendung von Sulfonylbenzyl-substituierten Pyridonen nach Anspruch 1 zur Herstellung von Arzneimitteln.

**10.** Verwendung von Sulfonylbenzyl-substituierten Pyridonen nach Anspruch 1 zur Herstellung von Arzneimitteln zur Behandlung von arterieller Hypertonie und Atherosklerose.